# EUROPEAN PATENT SPECIFICATION

(11) **EP 4 013 292 B1**
(45) Date of publication and mention of the grant of the patent: **31.12.2025**
(21) Application number: 20745215.2
(22) Date of filing: 29.07.2020
(51) Int. Cl.: A61B 5/00

(54) **APPARATUS FOR PROCESSING SENSOR SIGNALS**
VORRICHTUNG ZUR VERARBEITUNG VON SENSORSIGNALEN
APPAREIL DE TRAITEMENT DE SIGNAUX DE CAPTEUR

(30) Priority: 12.08.2019 WO PCT/EP2019/071632
(43) Date of publication of application: 22.06.2022
(73) Proprietor: Chronolife SAS, 75012 Paris (FR)
(72) Inventor: CHENEGROS, Guillaume, 89400 Bassou (FR); ROGER, Harmony, 77173 Chevry-Cossigny (FR); DUMAS, Pascal, 78000 Versailles (FR)
(74) Representative: Yes My Patent
(86) International application number: PCT/EP2020/071391
(87) International publication number: WO 2021/028223

(56) References cited:
- US-A1- 2007 299 325
- US-A1- 2015 047 091
- US-A1- 2016 374 615
- US-A1- 2018 024 622
- US-B1- 6 727 197

## Description

### Technical Field

The invention relates to the field of electronic devices embedded in textile materials, in particular a piece of clothing with an embedded apparatus for processing signals as well as methods for producing such an apparatus.

### Technological Background

Electronic devices for detecting and processing biometric signals of a user generally require that one or more detection units, or sensors, are in proximity of or even in direct contact with an anatomic region of a user. For example, one or more sensors may be applied to the skin of a user so as to perform a temperature measurement or determine physiological parameters. For example, a plurality of sensors may be applied to the chest area of a user so as to detect electrical signals related to a cardiac function of the user. Monitoring the various signals delivered by these sensors allows determining a user specific physiological condition that might be impaired. For example, when an individual is having a seizure, specific signal features appear on the signals corresponding to the electrocardiogram (ECG) or to respiration. To receive and process the signals and to determine such biometric parameters, the sensors are connected by a plurality of wires to an electronic device, which may be fixed to a garment or clothing item using e.g. one or more straps or via integrated push buttons.

However, the use of electronic devices in garments poses various problems. For example, external influences due to changing weather conditions or transpiration of the user may result in liquids accumulating at the electronic components, which may cause corrosion or a short-circuiting, eventually leading to a malfunctioning of the electronic device. To overcome such liquid penetration, a casing may be provided for one or more components, which may reduce or avoid such undesirable effects, but results in a bulky device and impairs the free movement of the user. Furthermore, the electronic device needs to be removed from the garment to allow the garment to be washed and to enable a charging or removal of the batteries of the electronic device, which may require the removal of the casing from the electronic device. Such removal may be impractical and may be accidentally forgotten prior to washing, e.g. in the case wherein the electronic device has relatively small dimensions, thereby resulting in a loss of functionality.

In addition, the wiring of the sensors may not be sufficiently held in place on the anatomic region of the user, which may be caused by the movement and/or the transpiration of the user. By the same token, the connecting region of the wires and the electronic device may be loosened due to the movement of the anatomic region, e.g. when a user is exercising, resulting in a loss of sensory signals and an incomplete determining of the corresponding biometric parameter.

US 2007/0299325 A1 discloses a physical status monitoring system. It includes a shirt and a stretchable circumferential band attached to the shirt. One or more sensors on the band are electrically connected to a signal transmission conductor.

US 2016/374615 A1 discloses a biosignal detecting garment including at least two electrodes, a measurement device, a wiring portion and a garment body on which the electrodes, the measurement device and the wiring portion are placed.

US 2018/0024622 A1 discloses a real-time motion capture system and garment.

Accordingly, there is a need for electronic devices and methods adapted for integration of the electronic device into a fabric and which reduce the above problems and which provide an improved connection of the electronic device with a textile without impairing the movement of a user.

### Summary of the invention

It is an object of the present invention to provide an apparatus for processing sensor signals having an electronic device and a textile interface which abrogates at least some of the above observations being undesirable for the user or implementation in a garment.

Accordingly, in a first aspect, an apparatus for processing sensor signals as defined by claim 1 is suggested. The apparatus comprises an electronic device comprising a rigid or flexible printed circuit board and configured to receive and process biometric sensor signals of a user and a plurality of electrically conducting pads connected to the electronic device, wherein each pad is connected to a respective electrically conducting wire configured for contacting a sensor corresponding to an anatomic region of a user and for providing a sensor signal to the electronic device and textile interface for use in a wearable fabric having a first and a second surface and comprising a porosity providing a fluidic communication between the first and second surface. According to a preferred embodiment, the pad is connected to a respective electrically conducting wire by sewing or similar means. According to preferred embodiment, this sewing is performed with the same conducting wire used for contacting one of the said sensors.

The electronic device and the plurality of pads are arranged on the first surface of a portion of the textile interface, and the plurality of conducting wires are arranged on the first surface of the textile interface. The electronic device, the plurality of pads and the plurality of conducting wires are attached to the textile interface. It should be noticed that according to a preferred embodiment, the conducting wires are sewn over the said textile interface, therefore each stitch might be present on both the first surface and the second surface.

The apparatus further comprises a coating material, which covers at least the electronic device, the plurality of pads and the portion of the textile interface and extends from the first surface to the second surface, thereby fluidically sealing and encapsulating at least the electronic device, the plurality of pads, and the portion of the textile interface.

The electronic device may be any device capable of processing sensor signals related to biometric parameters. For example, the electronic device may be a medical device configured to detect or record e.g. electrocardiogram (ECG), electroencephalogram (EEG), breathing pattern (including abdominal and costal patterns), lung impedance, body temperature, muscle activity or contraction, blood pressure and any other physiology signal. Accordingly, the electronic device may be part of a medical interface that enables patient measurements over prolonged time periods, e.g. for monitoring purposes or detection of physiological problems. By providing the conducting wires directly on the textile interface that may be integrated or embedded in a wearable fabric, a correct placement and contact with the appropriate sensor corresponding to an anatomic region may be ensured.

By the same token, the electronic device may also be configured as a wearable device to be worn during leisure or while performing sports. The electronic device may e.g. record particular movements and/or may record physiological parameters while exercising so as to evaluate the performance or monitor the user, e.g. to ensure that the user is exercising within predefined physiological boundaries.

According to preferred embodiment, the electronic device may also comprise an element for providing a required electrical energy e.g. a battery, preferably a welded battery.

In order to provide said measurements, the conducting wire may be configured to be coupled at a free end with a respective sensor and to receive and forward an electronic or optoelectronic signal, which may hence be received by a corresponding conducting pad. Accordingly, each conducting wire may be comprised of an electrically conducting material such as a metal, preferably a corrosive resistant metal. According to preferred embodiment, said conducting wire is comprised of fiber material (e.g. yarn), in particular a textile fiber, which is conductive. It generally comprises a base layer composed of a synthetic (e.g. polyamide) and/or natural fiber (e.g. cotton) combined with a conductive element formed, for example, of a plurality of nanoparticles or a permanently bonded silver layer. Ideally the selected conductive wire retains the flexibility and durability of the textile material. The conducting wire may have various dimensions and thicknesses so as to accommodate for different anatomical regions and biometric functions or to accommodate a sewing processing step. The number of conducting wires and conducting pads may vary depending on the required number of sensors and may range between 2 and 20, preferably between 5 and 13, to provide a number of accurate measurements while maintaining an average complexity of the control logic and while keeping the overall dimensions small.

The communication may either be one directional or bi-directional, such that the electronic device may only receive sensor signals or may be configured to both receive and output signals from and to the user, respectively. The electronic device comprises a control logic, e.g. in the form of a control unit and/or evaluation unit, which is connected to the conducting wires via the respective conducting pads, wherein the conducting pads may also be connected to each other.

Attachment of the various components to the textile interface may be provided e.g. by means of a preliminary or primary attachment with a fabric material or other material so as to hold the components at their respective predefined position on the textile interface. According to a preferred embodiment, the electronic device is attached to the textile interface at the conducting pad by sewing or similar means with conducting wire, preferably with electrically conducting yarn. According to a preferred embodiment, this sewing is performed with the same conducting wire or conductive yarn used for contacting with the sensors.

The final attachment may then be achieved by means of the coating material, which penetrates the textile interface and provides a continuous layer on both the second surface and the first surface of the textile interface and covering the electronic device, the battery when present, and the conducting pads. Thereby, the coating material provides an encapsulation of the electronic device, the battery when present, and conducting pads, which provides a material bonding or linkage and securely fixes the components to the textile interface. By the same token, the conducting wires may at least in part be covered with the coating material, e.g. at a connecting region extending from the conducting pads.

The coating has the advantage that a cover may be omitted and the thickness of the apparatus may be reduced, such that the thickness and hence bulkiness of the apparatus may be reduced and the apparatus may be easily implemented in a wearable fabric without impairing the movement of a user. Furthermore, the coating ensures that liquids may not penetrate the coating material, such that corrosion of the inner electronic components of the apparatus may be avoided. In addition, the direct coating increases the robustness by preventing a relative movement of the electronic components and by increasing the force distribution upon accidental impact. Thereby, the apparatus may also be machine washed without requiring a dismounting of particular components, such that a garment with an implemented apparatus does not require particular attention, which makes the product as a whole much more user friendly.

The coating material is hence a material that is not water-soluble and prevents penetration of liquids, e.g. water, so as to fluidically seal the electronic components. Furthermore, the coating material is preferably a non-conducting material to provide an electric isolation and to avoid the necessity of providing a primary coating with an electrically non-conducting material.

For easy implementation of the apparatus into a garment, the textile interface is preferably a flexible and/or resilient and/or elastic material, e.g. an elastomer or a poly(organo)siloxane (silicone) This not only provides that the textile is easy to handle, but may also conform to an anatomic region of a user. Accordingly, the borders or boundaries of the textile interface may e.g. be sewn into a wearable fabric and may be easily folded into the seams of a garment prior to finishing the garment. Thereby, the apparatus may be fully embedded into the wearable fabric and may hence not be visible to a user, thereby increasing the acceptance of the garment to a user.

In addition to the requirement of fluidically sealing the electronic components, and the battery when present, the coating material is preferably a flexible material, such that any movement of the apparatus is not significantly impaired by the coating or may result in a rupture of the continuous coating. The flexibility of the material may furthermore be experienced as more comfortable, when the apparatus is implemented in a clothing item and may provide a soft touch, preferably providing a resilience and low roughness. Preferably, the coating material comprises silicone or essentially consists of a silicone-based material. Such material allows a cost-effective encapsulation of the electronic components, a fast curing and an easy to handle processing while comprising a sufficient structural integrity and robustness for implementation in a clothing item and allowing, e.g., a machine washing.

The coating material may also comprise more than one layer, wherein each layer has been sequentially added after curing of the previous layer. The multiple layers may be based on the same material or may differ in their properties, wherein at least one layer ensures a fluidic sealing of the respective electronic components. The use of more than one layer may facilitate the initial adherence of the coating material to the respective electronic components and the textile interface, such that a primary fixation is established. Accordingly, this may facilitate the handling and processing of the apparatus and allows further layers to be added at a different time point and/or positioning of the apparatus.

Preferably, the porosity of the textile interface is provided by a plurality of holes at essentially equal spacing from each other. In other words, the textile interface may comprise an essentially homogeneous first and second surface. Preferably, the textile interface is a mesh material, most preferably tulle or a tulle-like material. The provision of the plurality of holes not only ensures that the coating material may easily penetrate the first and second surface and facilitate the distribution of the coating material, e.g. when the coating material is poured or sprayed onto the respective components of the apparatus, but also facilitates the implementation of the apparatus into a clothing item by ensuring that fixation may be provided at every region of the textile interface due to the homogenous distribution of the plurality of holes.

For example, the textile interface may simply be sewn into the seams of a clothing item or garment by means of the holes without impairing the structural integrity of the textile interface. At the same time, the plurality of holes ensures a full encapsulation of the respective electronic components. Thereby, the apparatus may be easily integrated or embedded into the clothing item while the respective electronic components are being protected from external influences. The use of a mesh or tulle material furthermore increases the breathability of the apparatus, such that the apparatus may also be implemented in e.g. sport gear or leisure equipment while at the same time reducing the overall weight of the apparatus.

Advantageously, each conducting wire is sewn into the textile interface, wherein the thread is the conducting wire and traverses the first and second surface of the textile interface. The attachment of the conducting wire may hence be primarily achieved by the sewing arrangement of the conducting wire. In order to provide such arrangement, the conducting wire may comprise e.g. a flexible material that allows bending and folding so as to orientate the conducting wire in various directions and to provide a sewing pattern for sufficient structural stability. For example, the conducting wire may comprise of one or more thin metal fibers that are sewn into the textile interface using conventional patterns and which together define the conducting wire. According to preferred embodiment, said conducting wire comprises conductive yarn that are sewn into the textile interface using conventional patterns and which together define the conducting wire. According to a preferred embodiment, said conductive yarn comprises metallic nanoparticles or metallic nanoparticles permanently bonded or plated metallic particles. According to a further preferred embodiment, said metallic particles or nanoparticles are silver particles.

The sewing engagement of the conducting wire with the textile interface hence achieves that the conducting wires are securely attached to the textile interface and do not require additional attachment means such as, e.g., mechanical fasteners or positive locking interfaces. To facilitate such sewing arrangement, the textile interface is preferably formed as a mesh or tulle material, such that the sewing does not significantly affect the structural stability of the textile interface and no additional holes have to be created in the textile interface.

As described in the above, the use of a mesh or tulle material furthermore has the advantage that the coating material may easily penetrate the first and second surface and facilitate the distribution of the coating material, e.g. when the coating material is poured or sprayed onto the respective components of the apparatus. Thereby, the covering of the conducting wires on each surface of the textile interface is facilitated. This is particularly the case when using a coating material that is flexible, such as silicone or a silicon-based material, which e.g. may be provided at a predefined viscosity during application to facilitate both the spreading and the adhesion of the coating material on both of the surfaces and the conducting wires. In addition, the use of such coating material may improve the haptics or tactile experience on both surfaces, also when the conducting wires engage the textile interface and traverse the first and second surface of the textile interface, since these materials may provide a soft touch and low roughness.

In addition, such fixation also provides that the conducting wires may be held at a predefined region, such that each conducting wire may receive electrical signals of said region via a corresponding sensor and the measurement accuracy and/or validity may be increased. In addition, mismatch or accidental misplacement of the conducting wires is avoided and user considerations, actions, and cognitive efforts may be reduced or are no longer required.

The arrangement of the conducting wires may depend on the type and the area for which measurements are to be obtained. Accordingly, the length and thickness of the conducting wires may be varied and the spacing between the conducting wires at the conducting pads, i.e. at the connecting region, may be essentially equal or may vary depending e.g. on the geometry and circuitry of the control logic of the electronic device.

Furthermore, each conducting pad may be secured to the textile interface by the plurality of conducting wires. For example, the conducting wires that are used as a thread may cover a portion of the electronic pads, e.g. one or more corners or edges of the respective electronic pad. This not only improves the connection between the respective conducting wire and electronic pad, but also increases the signal transduction due to a larger contacting surface.

To further improve the fixation of the conducting pads, each conducting pad preferably comprises at least one through hole and is sewn into the textile interface by the respective conducting wire extending through the at least one through hole. Thereby, each conducting wire may be connected to the respective conducting pad by at least one full loop, which secures the connection both between the conducting wire and the conducting pad and between the conducting pad and the textile interface. Furthermore, this avoids any relative movement between the conducting wires and the conducting pads.

The mechanical fixation of the conducting pads to the textile interface and the mechanical linkage between each conducting pad and the respective conducting wire may be further improved by providing additional holes in each conducting pad. Accordingly, each conducting pad preferably comprises two or three through holes. Thereby a more symmetrical and more profound fixation may be achieved, wherein a tilting of the respective conducting pad may be avoided and proper attachment of the connected conducting wire is hence also ensured during deformation of the apparatus, e.g. due to movement, for example when the apparatus is implemented in a clothing item.

Since the apparatus may be integrated in a clothing item, the apparatus may be sized and dimensioned accordingly. For example, it may be required that a height or thickness of the apparatus is kept small, so as to provide an essentially flat apparatus. To achieve such reduced height, each conducting wire may extend from the respective pad in a plane defined by the electronic device and the plurality of conducting pads. Accordingly, the conducting wires may have various orientations and lengths yet essentially remain in the same plane as the electronic device and conducting pads. In other words, the conducting wires may extend along the first and second surface of the textile interface yet do not extend in a direction perpendicular to said surfaces so as to avoid an increase in thickness of the apparatus.

The conducting wires may also be formed so as to accommodate or adapt to a variety of anatomic regions and movements of a user, which may not only have different curvatures, but also differ in the relative movement of the respective body part. Therefore, each conducting wire advantageously comprises an essentially straight portion, a curved portion, and/or a bent portion. For example, a straight portion may be advantageous in a situation where a straight movement is to be expected whereas a bent or curved portion may be advantageous in cases of an expected relative movement or bending of connected body parts or when an anatomic region comprises a curvature itself. Furthermore, bent or curved portions facilitate the detection of biometric signals at predefined positions without requiring a modification of the positioning of the corresponding conducting pads, such that the area of the apparatus that is provided with the coating material may be reduced.

Although a variety of conducting linkages may be provided between each conducting wire and the respective conducting pad, such linkage is preferably achieved by the conducting wire itself. Therefore, to ensure a full encapsulation of the conducting pads and the electronic device, and the battery when present, the coating material preferably covers a portion of each conducting wire connecting the conducting wire to the conducting pad. Thereby, a direct linkage is provided while at the same time a compact and fluidically secured configuration is achieved.

A compact design of the apparatus may also be achieved by using conducting pads that are integrated on the electronic device. The electronic device comprises a circuitry in the form of an electronic board or card, wherein the conducting pads are arranged at a predefined region. Thereby, the conducting pads are within the same plane as the electronic device, such that a thickness of the apparatus may be kept small. Furthermore, integration on the electronic device increases the structural integrity of the apparatus and reduces the occurrence of contact loss between the circuitry and the conducting pads.

Alternatively, the conducting pads may also be in direct contact with the textile interface and/or may be spaced apart from the electronic device. This results in an even more versatile arrangement of the electronic device and the conducting pads and further reduces the rigidity or bulkiness of the apparatus, allowing the apparatus to be more flexible, which may be advantageous when implementing the apparatus in a clothing item. Since the conducting pads are furthermore arranged on the same surface of the textile interface, said conducting pads are essentially also in the same plane as the electronic device. In such arrangement, the conducting pads may at least partially form an integrated circuit by means of connecting conducting wires, thereby forming a control logic, such that the electronic device may at least partially be substituted by the conducting pads and conducting wires. In other words, such arrangement may provide a "smart" textile and, depending on the configuration, may even fully omit the requirement of a separate or additional electronic device.

Preferably, the direct contact with the textile interface and/or the spaced apart arrangement is combined with a textile interface made of a mesh-like material to facilitate the arrangement and versatility of the apparatus, e.g., due to the homogenous structure. The mesh-like material furthermore facilitates the coating of the conducting wires and/or conducting pads, which is preferably provided by a silicon-based or other flexible coating material so as to improve the penetration of the first and second surface and facilitate the distribution of the coating material. In particular, such combination has the advantage that with a spaced apart arrangement encapsulation is facilitated at every region of the textile interface and fixation of the respective conducting pads may be improved. Thereby, the spaced apart arrangement also provides that the total consecutive area of the textile interface being encapsulated may be reduced, such that e.g. a more convenient wearing item may be provided that may better adapt to corresponding anatomical shapes and/or body movements.

The electronic device comprises a flexible printed circuit board or the electronic device comprises a rigid printed circuit board. Accordingly, the electronic device may be configured as an electronic board or electronic card with an integrated circuitry provided by conducting paths and comprising electronic components that are in communication with the plurality of conducting pads so as to process the received sensor signals. The implementation of a flexible printed circuit board further increases the conformity of the apparatus to an anatomic region of a user and allows a deformation not only during use, but also during e.g. machine washing, such that external forces acting on the apparatus do not impair the mechanical stability and integrity of the apparatus. In addition, the flexibility facilitates the integration into e.g. a clothing item since the textile interface may be handled more easily, for example, when sewn into seams of a clothing item or garment.

Since the electronic device may be sized and dimensioned to cover only a minimal area of the textile interface and a height may be minimized by avoiding mechanical fasteners or plugging arrangements, a relatively small rigid printed circuit board may be used as part of the electronic device. Such rigid PCB further ensures that material contacts and bonds are maintained and unsoldering events are avoided, even during machine washing. Thereby, the integrity of the apparatus may be further increased.

Due to the full encapsulation of the sensitive electronic compartments, the apparatus may be integrated in a secure fashion into a variety of clothing items and may function independent of external influences. The apparatus may hence be implemented in a wearable fabric, wherein the sensor signals received from respective sensors coupled to a free end of respective conducting wires provide a monitoring of biometric parameters of the user. To further increase the ease of use and independent functioning of the apparatus, the apparatus is preferably configured as portable stand-alone apparatus, wherein the electronic device comprises an electrical energy storage device connected to a charging interface.

Thereby, a user does not require any additional devices and may record biometric parameter data without any additional wiring or power supply unit, i.e. as a fully wireless implementation. The electrical energy storage device, e.g. a rechargeable battery or cell, hence provides the energy required to receive and process the sensor signals into biometric parameter data, such that the user may be provided with an overview or monitoring of the current or actual status of its physiological status. While the electrical energy storage device (i.e. battery) is also coated with the coating material, i.e. is within the encapsulation to ensure a fluidic sealing, the charging interface may be provided at a region of the textile interface that is not covered by the coating material, such that the encapsulated area may be kept small and the charging interface is directly accessible.

Preferably, the charging interface is formed as two connecting interfaces, which are each connected to the electronic device via a conducting wire sewn into the textile interface. Thereby, opposite charging terminals may be provided that are securely attached to the textile interface via the conducting wires. Furthermore, this allows that the same method of attachment may be implemented in securing the charging interface, which not only facilitates the manufacturing, but also increases the structural integrity and stability of the apparatus.

To further facilitate the charging and to provide an intuitive coupling of a charging device with the charging interface, each connecting interface preferably comprises an attachment means, preferably a hook-and-loop attachment or snap fastener. Accordingly, each of the charging terminals may only be coupled to a terminal of the charging device having a matching configuration, such that a mismatch and potential damage to the electrical energy storage device may be avoided. The charging interface may also be formed, at least in part, by conductive yarn.

As described in the above in view of the conducting pads, the electronic device may also comprise at least one through hole, wherein at least one conducting wire extends through said through hole and secures the electronic device to the textile interface. Thereby, the electronic device may be easily attached to the textile interface, for example, by means of the fixation of integrated conducting pads and one or more holes at opposing ends of the electronic device. Furthermore, this avoids any relative movement between the conducting wires and the electronic device.

More preferably, a conducting wire connecting the electrical energy storage device to the charging interface may extend through the additional one or more holes, such that no separate holes and conducting wires are required for securing the electronic device to the textile interface. This further increases the structural integrity of the electronic device and reduces the number of conducting wires and the overall weight and rigidity of the apparatus.

Although each conducting wire may be comprised of a plurality of conducting fibers, e.g. conductive yarn, or thin metal threads which together define a longitudinal shape of the conducting wire, this may not significantly affect the circumference of the combined plurality of fibers.

Accordingly, each conducting wire preferably comprises an essentially homogeneous outer surface and is formed of a plurality of conducting wires that are at least in part connected by material bonding. Such homogeneity may e.g. be considered as a core of the conducting wire with no protruding fibers in a radial direction and wherein the fibers arranged at an outer circumference are arranged essentially adjacently to each other, so as to minimize a space therebetween and provide an essentially smooth outer surface. Such smoothing may furthermore be increased by material bonding, which may be provided by means of local welding or soldering.

The apparatus may not only be configured to receive and process sensor signals, but preferably is also configured to process and store the biometric signals into multi-parameter biometrics data, wherein the apparatus may comprise a transmitter or transceiver for transmitting said data. Thereby, the monitoring may be provided by an external or remote device, which may give feedback to a user in the form of a visual representation, acoustic signal, and/or haptic or tactile feedback signal.

Preferably, the electronic device is configured to be wirelessly paired to a second device, in particular by reading out optical information attached to the apparatus on said second device. For example, the second device may be a smart phone or smart watch or computing device with a mobile interface, wherein the optical information may be present in the form of e.g. a scannable barcode or QR code. The second device then uses a camera or optical sensor for scanning said code and automatically provides a pairing between the second device and the electronic device of the apparatus, such that biometric data may be received from the apparatus. Thereby, the user, physician or medical personnel may quickly and easily couple or pair the apparatus with e.g. a wearable and/or mobile interface so as to monitor the biometric parameters on the second device, e.g. via Bluetooth, Zigbee, WLAN, NFC, RFID, or the like.

The invention further relates to a clothing item, preferably a T-shirt, according to claim 17 comprising a wearable fabric and an apparatus according to the invetion, wherein the textile interface of the apparatus is sewn into the wearable fabric, preferably at a region corresponding to the seams of the clothing item.

Accordingly, a fully wearable device is achieved, wherein the electronic device is embedded in the fabric of the clothing item. For example, the textile interface comprising the electronic components may be solidly sewn into a piece of tulle and comprise a coating comprising silicone, such that the apparatus comprises a high degree of flexibility, thereby facilitating the processing and integration of the apparatus in the clothing item.

This solution not only fully supports all body movements, but also allows multiple machine washes without requiring any modification or disassembly of the integrated apparatus. In other words, no plugging is needed during the use of the clothing item and the risk of detachment is significantly reduced due to the integrated structure and encapsulation, making the clothing item very easy-to-use. At the same time the presence of the electronic device is not apparent from the outside, thereby increasing the acceptance of a user.

Furthermore, by means of a charging interface, the electronic device may also be charged by means of a simple coupling to a power source, such that e.g. a battery does not need to be removed. For example, a charging may be provided by coupling a power source to charging terminals that are arranged on the textile interface outside of the encapsulation or may be performed by induction charging through the encapsulation material.

According to another aspect, a method of producing an apparatus for processing sensor signals according to claim 18 is suggested, comprising the following steps:
- providing an electronic device comprising a rigid or a flexible printed circuit board and configured to receive and process biometric sensor signals of a user and a plurality of electrically conducting pads connected to the electronic device, wherein each pad is connected to a respective electrically conducting wire configured for contacting a sensor corresponding to an anatomic region of a user and for providing a sensor signal to the electronic device;
- providing a textile interface for use in a wearable fabric having a first surface and a second surface and comprising a porosity providing a fluidic communication between the first surface and the second surface;
- arranging and attaching the electronic device and the plurality of pads on the first surface of a portion of the textile interface, and the plurality of conducting wires on the first surface of the textile interface; and
- providing a coating material over at least the electronic device and the plurality of pads and extending from the first surface through the textile interface to the second surface, sthereby fluidically sealing and encapsulating at least the electronic device, the plurality of pads and the portion of the textile interface.

According to a preferred embodiment, the biometric signals received from sensors are selected in the group of electrocardiogram (ECG), electroencephalogram (EEG), breathing pattern (including abdominal and costal patterns), lung impedance, body temperature, muscle activity or contraction, blood pressure and any other physiology signal.

The method hence allows a manufacturing of an apparatus that is fluidically sealed, thereby protecting the inner electronic components and, in particular, the electronic device. The coating hence replaces solid casings and plugging fixations, such that the electronic device forms a fully integrated feature of the textile interface and detachments of electronic components are essentially avoided.

The apparatus obtained by the method may generally correspond to the apparatus as described in the above, such that like features and technical advantages are not described again and it is instead referred to the description of the apparatus in the above.

To facilitate the arrangement and the attachment of the various components, the method may use a template printed on e.g. paper and using the desired dimensions of the apparatus. The textile interface may e.g. be at least semi-transparent and be arranged over the paper, wherein the paper and textile interface are cut along indicated lines that define the outer boundaries of the apparatus, which generally corresponds to the outer boundaries of the textile interface. The textile material may be folded around the paper material along a margin, such that the textile interface and paper template remain connected during further processing.

The electronic device, conducting pads, and conducting wires are then placed onto the first surface of the textile interface at the positions and according to the orientation indicated by the template and are attached to the textile interface. After attachment, the paper may be removed, e.g. by means of a forceps or tweezers. Subsequently, the electronic device and conducting pads are covered by a coating material, such that the coating material penetrates the textile interface and extends from the first surface to the second surface so as to fully cover said components at both sides, thereby forming an encapsulation. The coating may be provided by simple pouring or by spray coating, depending on the material and desired thickness of the coating.

The step of providing a coating material may include providing a first coating layer and a second coating layer over the first coating layer, wherein the coating material is preferably silicone-based. The first coating layer may e.g. be a relatively thin layer providing an initial cover of the electronic components, which facilitates the adherence of the second layer. The first and second coating layer may hence also be composed of different materials, but are preferably made of the same material so as to further facilitate the manufacturing. The second coating layer may then be applied after curing of the first coating layer and may comprise a greater thickness so as to provide a more homogeneous cover of the electronic components, which increases the structural stability and reduces the risk of detachment due to impact. In this regard silicone not only ensures that the electronic components are fluidically sealed, but also comprises a resilience upon deformation, such that forces acting upon the electronic components may be better distributed and damage to the electronic components is reduced or even avoided.

Alternatively, the step of providing a coating may include a step of injection molding with coating material according to well-known methods.

As described in the above, the textile interface is preferably a mesh material or tulle, wherein the conducting wire is attached to the textile interface by sewing using the conducting wire as a thread. The mesh material and, in particular, tulle provide a homogeneous surface, such that no further through holes are required and the existing holes may be used for sewing the conducting wires into the textile interface in the required orientation.

The conducting wires may be formed of conducting fibers or a lead wire that are used as a thread and may be inserted and used in a sewing machine, wherein the sewing needle performs the sewing. This may require that the sewing machine is initially configured according to the requirements of the attachment and the characteristics of the textile interface, e.g. the spacing between the respective holes (for example about 0,25 cm), such that further punctures and or wrinkles of the textile interface may be avoided. The lead wire may hence be provided on a role so as to provide a continuous segment that may traverse the textile interface and forms a conducting wire based on a plurality of fiber segments that define a core and a circumference based on the number of fibers. Any protruding fibers may furthermore be either removed or be welded or soldered by moving the welding or soldering iron along the outer circumference of the conducting wire, thereby achieving a material bonding of the plurality of fibers.

To facilitate the attachment of the conducting pads, each conducting pad preferably comprises at least two through holes, wherein the step of attaching the conducting pads to the textile interface preferably comprises sewing each contact pad into the into the textile interface using the respective conducting wire as a thread and by extending the conducting wire through the through holes. The through holes may be provided in advance, e.g. by stamping, punching, die-cutting or, preferably, by the sewing needle of the sewing machine. Alternatively, the holes may be formed during the sewing advancement, such that the sewing needle forms the hole while at the same time extending the conducting lead wire through the hole during the sewing movement.

The conducting pads may be integrated on the electronic device or may be in direct contact with the textile interface and/or be spaced apart from the electronic device. Accordingly, the conducting pads may e.g. be sewn into the textile interface independent of the electronic device so as to form a plurality of interconnected contact pads that together may form a control logic or "smart" textile interface. However, the conducting pads may also be integrated on the electronic device, for example, as integrated components on a (rigid) printed circuit board.

As for the conducting pads, the electronic device preferably comprises at least one through hole, wherein the step of attaching the electronic device to the textile interface preferably comprises sewing the electronic device using said through hole and using a conducting wire as a thread. Thereby, an attachment of the electronic device to the textile interface may be provided using the same sewing technique, such that the electronic device is integrated into the textile interface and separation is avoided without requiring e.g. plugging arrangements or mechanical fasteners or the like. The attachment is furthermore independent of the coating or encapsulation, such that the stress provided on said coating may be reduced.

The above method may advantageously be implemented in the production or manufacturing of producing a clothing item, preferably a T-shirt according to claim 24, comprising the above steps and by providing a wearable fabric, wherein the textile interface is sewed into the wearable fabric, preferably at a region corresponding to the seams of the piece of clothing. The textile interface, e.g. a flexible tulle material, carrying the electronic components may hence be folded into the seams of a garment at the required position to obtain sensor signals related to biometric parameters from a corresponding anatomic region when wearing the garment. The textile interface allows an easy integration into the wearable fabric, such that the wearable fabric may conceal a significant portion of the apparatus, thereby not significantly affecting the overall appearance of the garment.

### Brief description of the drawings

The present disclosure will be more readily appreciated by reference to the following detailed description when being considered in connection with the accompanying drawings in which:
Figure 1 is a schematic top view representation of an apparatus according to the invention having a textile interface prosthetic;
Figure 2 is a schematic top view representation of an apparatus according to Figure 1 with a particular type of textile interface and an alternative arrangement of conducting wires;
Figures 3 is a cross sectional view of the apparatus according to Figure 2 along line A-A;
Figures 4 is a cross sectional view of the apparatus according to Figure 3 implemented in a clothing item; and
Figures 5A and 5B show a schematic top view of an apparatus similar to the embodiment according to Figure 1 with an alternative arrangement of the conducting wires.

### Detailed description of preferred embodiments

In the following, the invention will be explained in more detail with reference to the accompanying figures. In the Figures, corresponding elements are denoted by identical reference numerals and repeated description thereof may be omitted in order to avoid redundancies.

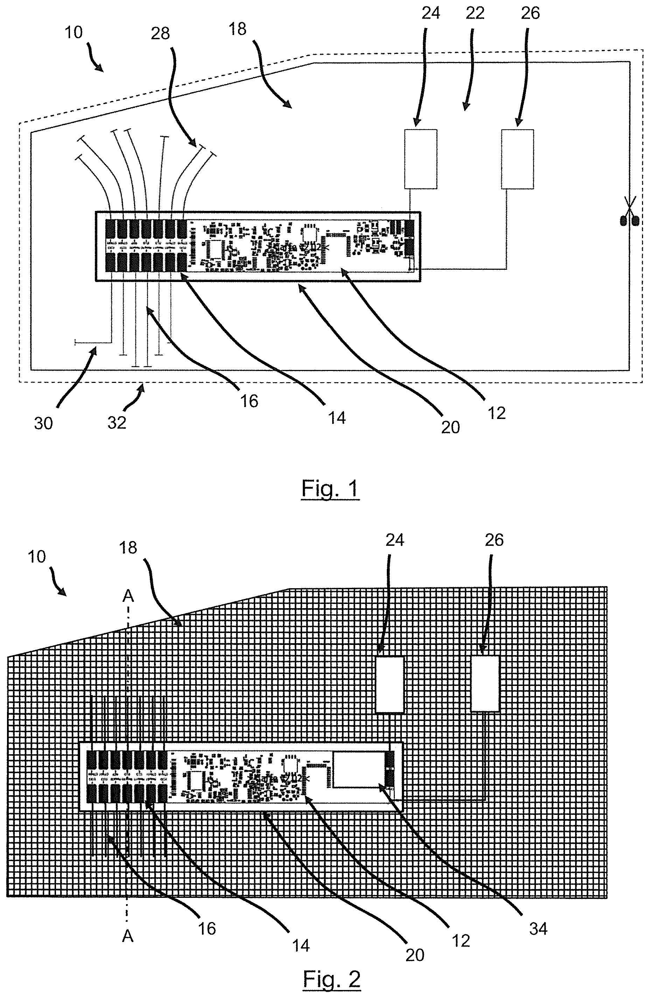

In Figure 1 a schematic depiction of an apparatus 10 according to the invention is shown in a top view. The apparatus 10 comprises a textile interface 18 having a first surface and a second surface, wherein only the first surface is visible from the top. The textile interface 18 serves as a substrate, such that various components may be held by the textile interface 18. The borders or boundaries of the textile interface 18 are indicated with a solid line, which during manufacturing may be printed on a paper template to indicate the required dimensions and shape. In such case, the textile interface 18 may be formed of an at least partially transparent material and placed onto the paper template indicating a predefined arrangement according to scale. The solid line is furthermore depicted with a scissors symbol to indicate that the textile interface is to be cut along this line. Furthermore, margins 32 indicated by the dashed line indicate the boundary of the template, such that the textile interface 18 may e.g. be folded into or over said margin 32, if required.

The textile interface 18 according to the embodiment is made of a flexible material that comprises a predefined porosity to allow a fluid communication between the first surface and second surface (not shown). However, the textile interface 18 itself is preferably not readily deformable, such that it provides a substrate having sufficient rigidity and structural integrity carry various components. Accordingly, an electronic device 12 is placed onto the textile interface 18, which is optionally configured as a printed circuit board with an integrated microprocessor and an integrated circuitry in the form of connecting conducting paths. The printed circuit board is optionally formed of a small-sized rigid printed circuit board, such that the integration of the electronic device 12 into the textile interface 18 does not adversely affect the flexibility of the textile interface 18, thus facilitating the handling and further processing of the textile interface 18.

The electronic device 12 is configured to process biometric signals of a user and may optionally be configured as a health monitor or as a part of a medical device. In order to process such signals, the electronic device 12 is connected with a plurality of integrated electrically conducting pads 14 that are each connected to a respective electrically conducting wire 16, as shown on the left of Figure 1. The conducting wires 16 are hence linked to the conducting pads 14 and extend directly from said pads 14. Accordingly, when the textile interface 18 is brought into contact with an anatomic region of a user, e.g. when the textile interface 18 and the apparatus 10 as a whole is integrated in a clothing item, the conducting wires 16 may be coupled with respective sensors provided on the clothing item, e.g. by contacting a respective sensor with a free end of a respective conducting wire 16, such that electric signals may be received by the conducting wires 16 and transduced to the electronic device 12 via the conducting pads 14.

To facilitate that electric signals may be continuously obtained, the conducting wires 16 may furthermore comprise one or more curves or bent portions so as to provide a curved conducting wire 28 or bent conducting wire 30. This not only ensures an optimized contacting surface or matching to the anatomic region, but also ensures that the electric signals are received from a predefined region and furthermore exhibit a predefined spacing. It will be obvious that the number of conducting wires 16, 28, 30 and the number of pads 14 as well as the respective arrangements and orientations are merely exemplary.

The apparatus 10 furthermore comprises a coating material 20, which is also indicated with a solid line and encloses the electronic device 12 and the conducting pads 14. The coating material 20 fully covers the electronic device 12 and the conducting pads 14 and furthermore covers a connecting region of the conducting wires 16, i.e. at an end extending from the conducting pads 14. This ensures that the conducting wires 16 may also at least partly be fixed by the coating material 20 and relative movement between the conducting wires 16 and the conducting pads 14 is prevented.

The coating material 20 hence forms an encapsulation for the sensitive electronic components of the apparatus 10. Although not shown in Figure 1, the coating material 20 extends from the first or top surface to the second or bottom surface due to the porosity of the textile interface 18. Thereby, a full fluidic sealing is provided. In the embodiment, the coating material 20 is composed of a silicone material, which offers some degree of resilience and is easy to apply and process while ensuring that no liquids may penetrate the coating material 20 into the electronic device 12. The coating material 20 is depicted as a transparent material, however, other optical properties may be chosen, e.g. via material selection and/or coloring agents, such that the electronic components may also be concealed and/or the color may match the textile interface 18 and/or a wearable fabric into which the textile interface is integrated at a later stage.

The encapsulation provided by the coating material 20 hence results in a light weight yet robust apparatus 10, which omits the use of any mechanical fixations and casings, such that the apparatus 10 comprises a reduced thickness and does not require any plugging for attaching the electronic device 12 to the textile interface 18.

Although a variety of methods may be used to attach the conducting wires 16, the conducting pads 14 and the electronic device 12 to the textile interface 18, the embodiment according to Figure 1 has conducting wires 16, 28, 30 that are sewn into the textile interface 18, wherein the thread is the conducting wire 16, 28, 30 and which traverses the first and second surface of the textile interface 18. Accordingly, a sewing pattern may be achieved using a conducting lead wire, so as to form respective conducting wires 16, 28, 30 formed of a plurality of conducting segments or fibers, e.g. conductive yarn. Using such arrangement and attachment, no further attachment means are required while at the same time this ensures that the electronic components of the apparatus 10 are securely attached to each other, such that any separation or detachment may be avoided. While the conducting wires 16, 28, 30 are intertwined with the textile interface 18, the conducting pads 14 and the electronic device 12 may comprise one or more through holes through which the conducting wires 16, 28, 30 extend, e.g. via a closed loop.

For providing a required electrical energy to the electronic device 12, the apparatus 10 may furthermore comprise e.g. a battery. Such battery (not shown) may be integrated in the electronic device 12, wherein a charging interface 22 arranged outside of the encapsulation may be connected to the battery via respective conducting wires. Accordingly, the battery may be charged using the first and second charging terminal 24, 26, which are connected to the battery via corresponding conducting plates or pads, indicated on the right of the electronic device 12 by the black rectangles.

Thereby, the apparatus 10 may function fully independently and may be easily integrated into e.g. a wearable fabric so as to provide a "smart" clothing item.

To further facilitate the encapsulation of the electronic components and to provide an easier handling of the textile interface 18, the textile interface 18 may be formed of a mesh material, such as tulle, as depicted in the embodiment according to Figure 2. Accordingly, a flexible material is provided having a homogeneous structure with sufficient structural integrity to be used in clothing implementations. Tulle has the advantage that it increases the acceptance by a user and increases the breathability due to the continuous arrangement of holes. Furthermore, this significantly reduces effort when integrating the textile interface 10 in a wearable fabric of a clothing item since the tulle may be bent and folded as desired, depending on the clothing characteristics and boundaries. In addition, due to the plurality of holes in all directions no further holes or punctures in the textile interface 18 are required while at the same time the degree of flexibility of arranging and attaching the respective components is greatly enhanced.

In Figure 2 a battery 34 is furthermore depicted as an integrated component of the electronic device 12. In addition, an alternative arrangement of the conducting wires 16 is provided. This provides a more compact design of the apparatus, which may be desirable if e.g. an equal spacing between the conducting wires 16 is required and no relative movements or curvatures of anatomic regions are expected in the area wherein the apparatus 10 is applied. However, it will be obvious that the such arrangement is not limiting.

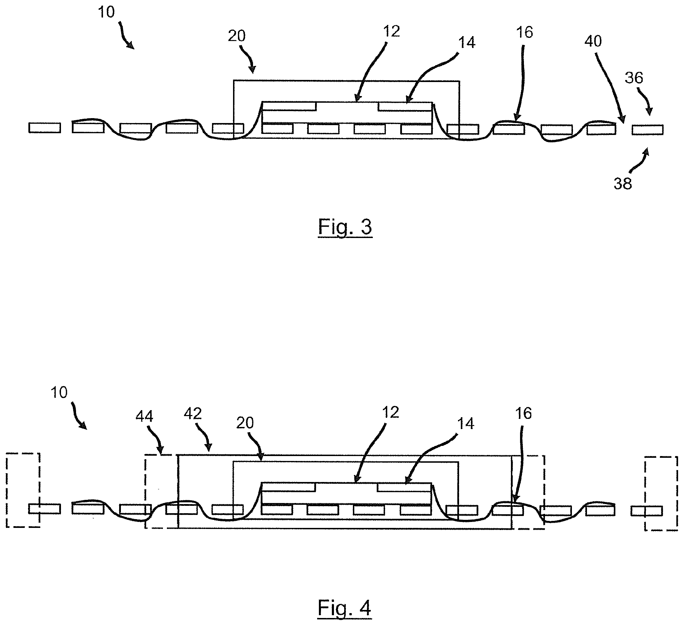

A cross-sectional view of the apparatus 10 along the line A-A depicted in Figure 2 is shown in Figure 3. Here, a more detailed schematic view of the arrangement of the various components of the apparatus is provided. Accordingly, the conducting wires 16 extend from the conducting pads 14 in a single plane with the electronic device 12 and the textile interface 18, as also follows from the top view in Figures 1 and 2. Furthermore, it is shown that the electronic components are placed on the first surface 36 of the textile interface 18 while the coating material 20 extends from the first surface 36 through the textile interface 18 to the second surface 38 so as to form an encapsulation of the electronic device 12 and the conducting pads 14. In addition, the encapsulation also includes a connecting region of the conducting wires 16, i.e. at a portion where the conducting wires originate from the conducting pads 14.

The attachment of the conducting pads 14 and the electronic device 12 via the conducting wires 16 is not shown in detail, however, it may be envisaged that each of said components comprises one or more through holes, e.g. two or three holes, through which the conducting wire 16 extends.

Such attachment, i.e. a sewing attachment is schematically depicted by the traversing of the conducting wires 16 through the holes 40, so as to form an attachment and connection with the textile interface 18. Following essentially the same plane as the conducting pads 14, the textile interface 18, and the electronic device 12, the conducting wires 16 do not significantly protrude in a direction perpendicular to the first and second surface 36, 38 and hence form a smooth surface. Although the specific pattern may vary, it is generally contemplated that the sewing arrangement comprises both forward and backward stitches so as to ensure that the conducting wire 16 may not accidentally be loosened during prolonged use.

In Figure 4 an embodiment is shown wherein the apparatus 10 according to Figure 3 is sewn into a clothing item. Accordingly, the textile interface 18 is integrated in a wearable fabric 40 by sewing the textile interface 18 into seams 42 of the wearable fabric 40, which are indicated by the dashed lines. This results in an embedded electronic device 12 in a garment or clothing piece, wherein a portion of the conducting wires 16 may not be covered by either the textile interface 18 or the wearable fabric 40 and forms a free end, which may be exposed, brought into contact with, or coupled to a sensor arranged at the wearable fabric 40 and corresponding to an anatomic region of a user, when wearing the clothing item. Thereby, the conducting wires 16 may obtain signals, e.g. electric signals related to physiological parameters, which are transduced to the conducting pads 14 and are received by the electronic device 12, wherein the electronic device 12 processes said signals and determines one or more biometric parameter values therefrom.

The determined biometric parameters and corresponding values may then e.g. be transmitted to a coupled second device via a transmitter arranged on the electronic device 12 to provide a monitoring of a physiological status or health status of the user. Alternatively, or in addition, the electronic device 12 may comprise e.g. a signal outputting device such as an electromechanical transducer or buzzer to alarm a user when the determined parameter value exceeds a physiological boundary. Thereby, various arrangements may be provided to process the sensor signals and provide a feedback to the user or e.g. a physician, physiotherapist, or sport coach.

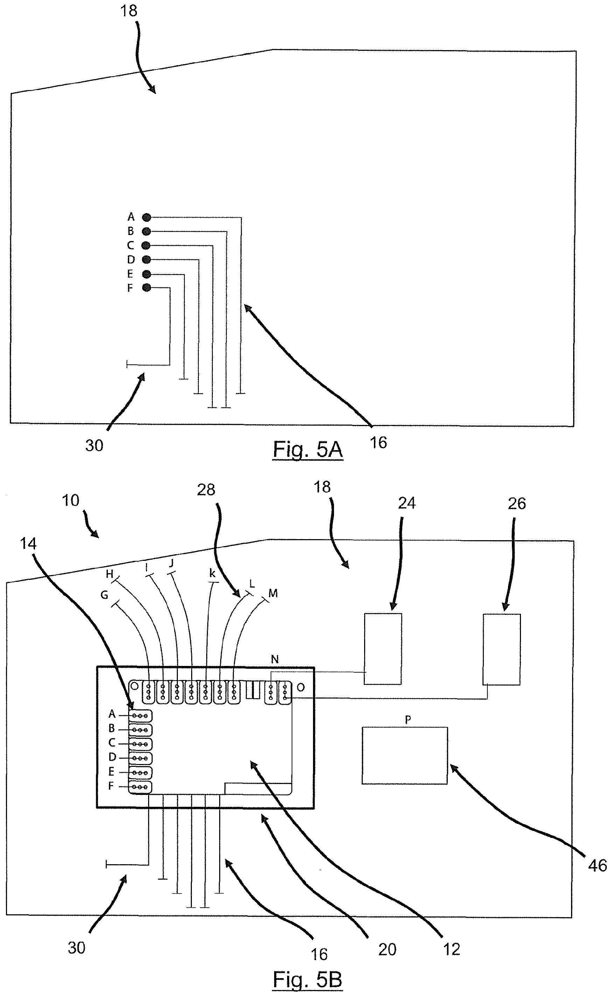

Figures 5A and 5B show a schematic top view of an apparatus 10 similar to the embodiment according to Figure 1 with an alternative arrangement of the conducting wires 16. In this embodiment, the conducting wires 16 are arranged either below the electronic device 12 (A to F) or on top of the electronic device 12 (G to M), such that the conducting wires 16 arranged underneath the electronic device 12, i.e. conducting wires A-F, are to be attached to the textile interface 18 before placing the electronic device 12 on the textile interface 18. Accordingly, Figure 5A depicts the textile interface 18, wherein the conducting wires A-F are securely attached to the textile interface 18 using a sewing technique, such that the conducting wires A-F extend in an essentially straight fashion as indicated with the reference numeral 16 or in a bent fashion, as indicated by the reference numeral 32. The thick dot indicates both the starting point and end point of the sewing thread or conducting wire.

From each respective starting and end point, a free end extends from the textile interface 18, which is intended for attachment to the corresponding conducting pad 14, as shown in Figure 5B. Accordingly, the free ends are pulled away from the textile interface 18 and are held towards the left of the textile interface 18 prior to placing the electronic device 12 on the textile interface 18 and the conducting wires A-F. Thereby, a short-circuiting is prevented and the free ends of the respective conducting wires A-F may be attached to the conducting pads 14 via the through holes of the conducting pads 14 and by using the same sewing technique.

After attachment of the conducting wires A-F to the conducting pads 14, the conducting wires G-M are attached to the respective conducting pads 14 and are attached to the textile interface 18 by a corresponding pattern, providing curved conducting wires 28. The first and second charging terminals 24, 26 may then be connected to the textile interface 18 and the electronic device 12 and the coating material 20 may then be applied to the textile interface 18 and the electronic device 12 as described in the above.

Furthermore, an identification tag 46 may be attached to the textile interface 18, which allows an identification of the electronic device 12 after applying the coating material 20 and/or implementation in a clothing item. Such identification may provide a coupling with an external device and/or may perform a calibration or installation via wireless transmission, such that the apparatus may form an interface for a user providing biometric parameter data according to a particular configuration. For example, the identification tag 46 may be a scannable object such as a QR-coded label, which may be read by an optical reader, e.g. integrated in a mobile terminal or portable device so as to provide a coupling between the apparatus and the portable device.

It will be obvious for a person skilled in the art that these embodiments and items only depict examples of a plurality of possibilities.

### List of reference numerals

- 10: Apparatus
- 12: Electronic device
- 14: Conducting pad
- 16: Conducting wire
- 18: Textile interface
- 20: Coating material
- 22: Charging interface
- 24: First charging terminal
- 26: Second charging terminal
- 28: Curved conducting wire
- 30: Bent conducting wire
- 32: Margin
- 34: Battery
- 36: First surface
- 38: Second surface
- 40: Through hole
- 42: Wearable fabric
- 44: Seam
- 46: Identification tag

## Claims

1. Apparatus (10) for processing sensor signals, comprising:
- an electronic device (12) comprising a rigid or a flexible printed circuit board and configured to receive and process biometric sensor signals of a user,
- a plurality of electrically conducting pads (14) connected to the electronic device (12), wherein each pad (14) is connected to a respective electrically conducting wire (16, 28, 30; A-M) configured for contacting a sensor corresponding to an anatomic region of a user and for providing a sensor signal to the electronic device (12), and
- a textile interface (18) for use in a wearable fabric (42) having a first surface (36) and a second surface (38) and comprising a porosity providing a fluidic communication between the first surface (36) and the second surface (38), wherein the electronic device (12)and the plurality of pads (14) are arranged on the first surface (36) of a portion of the textile interface (18), and the plurality of conducting wires (16, 28, 30; A-M) are arranged on the first surface (36) of the textile interface (18) and wherein the electronic device (12), the plurality of pads (14) and the plurality of conducting wires (16, 28, 30; A-M)) are attached to the textile interface (18),
wherein the apparatus (10) further comprises a coating material (20) covering at least the electronic device (12), the plurality of pads (14), and the portion of the textile interface (18) and extending from the first surface (36) through the textile interface (18) to the second surface (38), thereby fluidically sealing and encapsulating at least the electronic device (12), the plurality of pads (14) and the portion of the textile interface (18).

2. Apparatus (10) according to claim 1, wherein the electronic device (12) further comprises a battery for providing electric energy to the electronic device (12).

3. Apparatus (10) according to claim 1 or 2, wherein the coating material (20) is a flexible material, preferably comprising silicone,
and/or
wherein the coating material (20) is molded over at least the electronic device (12) and the plurality of pads (14).

4. Apparatus (10) according to any of the preceding claims, wherein the porosity of the textile interface (18) is provided by a plurality of holes (40) at essentially equal spacing from each other,
wherein the textile interface (18) is preferably a mesh material, more preferably tulle.

5. Apparatus (10) according to any of the preceding claims, wherein each conducting wire (16, 28, 30; A-M) is sewn into the textile interface (18), wherein the thread is the conducting wire (16, 28, 30; A-M) traversing the first and second surface (36, 38) of the textile interface (18).

6. Apparatus (10) according to claim 1, wherein the coating material (20) is a flexible material and wherein the porosity of the textile interface (18) is provided by a plurality of holes (40) at essentially equal spacing from each other, said textile interface (18) being a mesh material, and wherein each conducting wire (16, 28, 30; A-M) is sewn into the textile interface (18), wherein the thread is the conducting wire (16, 28, 30; A-M) traversing the first and second surface (36, 38) of the textile interface (18),
preferably wherein the coating material comprises silicone or is silicone-based and/or
wherein the textile interface is made of tulle.

7. Apparatus (10) according to any one of claims 5 or 6, wherein each of the conducting pads (14) is secured to the textile interface (18) by a respective one of the plurality of conducting wires (16, 28, 30; A-M),
preferably wherein each conducting pad (14) comprises at least one through hole and is sewn into the textile interface (18) by the respective conducting wire (16, 28, 30; A-M) extending through the at least one through hole,
wherein each conducting pad (14) more preferably comprises two or three through holes.

8. Apparatus (10) according to any of the preceding claims, wherein each conducting wire (16, 28, 30; A-M) extends from the respective pad (14) in a plane defined by the electronic device (12) and the plurality of conducting pads (14),
and/or
wherein each conducting wire (16, 28, 30; A-M) comprises an essentially straight portion, a curved portion (28), and/or a bent portion (30).

9. Apparatus (10) according to any of the preceding claims, wherein the coating material (20) covers a portion of each conducting wire (16, 28, 30; A-M) connecting the conducting wire (16, 28, 30) to the conducting pad (14).

10. Apparatus (10) according to any of the preceding claims, wherein (i) the conducting pads (14) are integrated on the electronic device (12),
or
wherein (ii) the conducting pads are in direct contact with the textile interface and/or are spaced apart from the electronic device,
preferably wherein (a) the conducting pads are in direct contact with the textile interface and are spaced apart from the electronic device.

11. Apparatus (10) according to claim 10 (ii)(a), wherein the coating material (20) is a flexible material, wherein the porosity of the textile interface (18) is provided by a plurality of holes (40) at essentially equal spacing from each other, said textile interface (18) being a mesh material, and wherein each conducting wire (16, 28, 30; A-M) is sewn into the textile interface (18), wherein the thread is the conducting wire (16, 28, 30; A-M) traversing the first and second surface (36, 38) of the textile interface (18) and wherein the conducting pads are in direct contact with the textile interface and are spaced apart from the electronic device.

12. Apparatus (10) according to any of the preceding claims, wherein the electronic device (12) comprises a rigid printed circuit board.

13. Apparatus (10) according to any of the preceding claims, wherein the apparatus (10) is configured as portable stand-alone apparatus and wherein the electronic device (12) comprises an electrically energy storage device (34) connected to a charging interface (22),
wherein the charging interface (22) is preferably formed as two connecting interfaces (24, 26), each connected to the electronic device (12) via a conducting wire (16, 30) sewn into the textile interface (18),
wherein each connecting interface (24, 26) more preferably comprises an attachment means, even more preferably a hook-and-loop attachment or snap fastener.

14. Apparatus (10) according to any of the preceding claims, wherein the electronic device (12) comprises at least one through hole and wherein at least one conducting wire extends through said through hole and secures the electronic device (12) to the textile interface (18).

15. Apparatus (10) according to any of the preceding claims, wherein each conducting wire (16, 28, 30; A-M) comprises an essentially homogeneous outer surface and is formed of a plurality of conducting wires that are at least in part connected by material bonding.

16. Apparatus (10) according to any of the preceding claims, wherein the electronic device (12) is configured to receive, process and store the biometric sensor signals into multi-parameter biometrics data and comprises a transmitter or transceiver for transmitting said data,
wherein the electronic device (12) is preferably configured to be wirelessly paired to a second device, more preferably by reading out optical information attached to the apparatus (10) on said second device.

17. Clothing item, preferably a T-shirt, comprising a wearable fabric (42) and an apparatus (10) according to any of the preceding claims, wherein the textile interface (18) of the apparatus is sewn into the wearable fabric (42), preferably at a region corresponding to the seams (44) of the clothing item.

18. Method of producing an apparatus for processing sensor signals, comprising the steps of:
- providing an electronic device comprising a rigid or a flexible printed circuit board and configured to receive and process biometric sensor signals of a user, and a plurality of electrically conducting pads connected to the electronic device, wherein each pad is connected to a respective electrically conducting wire configured for contacting a sensor corresponding to an anatomic region of a user and for providing a sensor signal to the electronic device;
- providing a textile interface for use in a wearable fabric having a first surface and a second surface and comprising a porosity providing a fluidic communication between the first surface and the second surface;
- arranging and attaching the electronic device and the plurality of pads on the first surface of a portion of the textile interface, and the plurality of conducting wires on the first surface of the textile interface; and
- providing a coating material over at least the electronic device and the plurality of pads and extending from the first surface through the textile interface to the second surface, thereby fluidically sealing and encapsulating at least the electronic device, the plurality of pads and the portion of the textile interface.

19. Method according to claim 18, wherein the electronic device provided further comprises a battery for providing electric energy to the electronic device (12).

20. Method according to claim 18 or 19, wherein (i) the step of providing a coating material includes injection molding with coating material, wherein the coating material is preferably an elastomer or more preferably silicone-based,
and/or
wherein (ii) the textile interface is a mesh material or tulle and wherein the conducting wire is attached to the textile interface by sewing using the conducting wire as a thread.

21. Method according to any of claims 18 to 20, wherein the method provides an apparatus according to any of claims 1 to 16.

22. Method according to claim 20 (ii) or 21, wherein each conducting pad comprises at least two through holes and wherein the step of attaching the conducting pads to the textile interface comprises sewing each contact pad into the into the textile interface using the respective conducting wire as a thread and by extending the conducting wire through the through holes,
preferably wherein the conducting pads are integrated on the electronic device or wherein the conducting pads are in direct contact with the textile interface and/or are spaced apart from the electronic device.

23. Method according to any of claims 18 to 22, wherein the electronic device comprises at least one through hole and wherein the step of attaching the electronic device to the textile interface comprises sewing the electronic device using said through hole and using a conducting wire as a thread.

24. Method of producing a piece of clothing, preferably a T-shirt, comprising the steps of the method according to any of the preceding claims 18 to 23 and providing a wearable fabric and sewing the textile interface into the wearable fabric, preferably at a region corresponding to the seams of the piece of clothing.

## Patentansprüche

1. Einrichtung (10) zum Verarbeiten von Sensorsignalen, umfassend:
- eine elektronische Vorrichtung (12), die eine starre oder eine flexible Leiterplatte umfasst und konfiguriert ist, um biometrische Sensorsignale eines Benutzers zu empfangen und zu verarbeiten,
- eine Vielzahl von elektrisch leitfähigen Pads (14), die mit der elektronischen Vorrichtung (12) verbunden sind, wobei jedes Pad (14) mit einem jeweiligen elektrisch leitfähigen Draht (16, 28, 30; AM) verbunden ist, das zum Kontaktieren eines Sensors, der sich mit einem anatomischen Bereich eines Benutzers deckt, und zum Bereitstellen eines Sensorsignals an die elektronische Vorrichtung (12) konfiguriert ist, und
- eine Textilschnittstelle (18) zur Verwendung in einem tragbaren Gewebe (42), das eine erste Oberfläche (36) und eine zweite Oberfläche (38) aufweist und eine Porosität umfasst, die eine Fluidkommunikation zwischen der ersten Oberfläche (36) und der zweiten Oberfläche (38) bereitstellt, wobei die elektronische Vorrichtung (12) und die Vielzahl von Pads (14) auf der ersten Oberfläche (36) eines Abschnitts der Textilschnittstelle (18) angeordnet sind und die Vielzahl von leitfähigen Drähten (16, 28, 30; A-M) auf der ersten Oberfläche (36) der Textilschnittstelle (18) angeordnet sind und wobei die elektronische Vorrichtung (12), die Vielzahl von Pads (14) und die Vielzahl von leitfähigen Drähten (16, 28, 30; A-M)) an der Textilschnittstelle (18) befestigt sind,
wobei die Einrichtung (10) ferner ein Beschichtungsmaterial (20) umfasst, das mindestens die elektronische Vorrichtung (12), die Vielzahl von Pads (14) und den Abschnitt der Textilschnittstelle (18) bedeckt, und und sich von der ersten Oberfläche (36) durch die Textilschnittstelle (18) zu der zweiten Oberfläche (38) erstreckt, wodurch mindestens die elektronische Vorrichtung (12), die Vielzahl von Pads (14) und der Abschnitt der Textilschnittstelle (18) fluidisch abgedichtet und eingekapselt werden.

2. Einrichtung (10) nach Anspruch 1, wobei die elektronische Vorrichtung (12) ferner eine Batterie zum Versorgen der elektronischen Vorrichtung (12) mit elektrischer Energie umfasst.

3. Einrichtung (10) nach Anspruch 1 oder 2, wobei das Beschichtungsmaterial (20) ein flexibles Material ist, vorzugsweise umfassend Silikon,
und/oder
wobei das Beschichtungsmaterial (20) mindestens über die elektronische Vorrichtung (12) und die Vielzahl von Pads (14) geformt wird.

4. Einrichtung (10) nach einem der vorstehenden Ansprüche, wobei die Porosität der Textilschnittstelle (18) durch eine Vielzahl von Löchern (40) in im Wesentlichen gleichem Abstand voneinander bereitgestellt wird,
wobei die Textilschnittstelle (18) vorzugsweise ein Netzmaterial, besonders bevorzugt Tüll, ist.

5. Einrichtung (10) nach einem der vorstehenden Ansprüche, wobei jeder leitfähige Draht (16, 28, 30; A-M) in die Textilschnittstelle (18) eingenäht ist, wobei der Faden der leitfähige Draht (16, 28, 30; A-M) ist, der die erste und die zweite Oberfläche (36, 38) der Textilschnittstelle (18) durchquert.

6. Einrichtung (10) nach Anspruch 1, wobei das Beschichtungsmaterial (20) ein flexibles Material ist und wobei die Porosität der Textilschnittstelle (18) durch eine Vielzahl von Löchern (40) in im Wesentlichen gleichem Abstand voneinander bereitgestellt wird, wobei die Textilschnittstelle (18) ein Netzmaterial ist, und wobei jeder leitfähige Draht (16, 28, 30; A-M) in die Textilschnittstelle (18) eingenäht ist, wobei der Faden der leitfähige Draht (16, 28, 30; A-M) ist, der die erste und die zweite Oberfläche (36, 38) der Textilschnittstelle (18) durchquert,
vorzugsweise wobei das Beschichtungsmaterial Silikon umfasst oder silikonbasiert ist und/oder wobei die Textilschnittstelle aus Tüll besteht.

7. Einrichtung (10) nach einem der Ansprüche 5 oder 6, wobei jedes der leitfähigen Pads (14) durch einen jeweiligen der Vielzahl von leitfähigen Drähten (16, 28, 30; A-M), an der Textilschnittstelle (18) befestigt ist,
wobei vorzugsweise jedes leitfähige Pad (14) mindestens ein Durchgangsloch umfasst und durch den jeweiligen leitfähigen Draht (16, 28, 30; AM), der sich durch das mindestens eine Durchgangsloch erstreckt, in die Textilschnittstelle (18) eingenäht ist,
wobei jedes leitfähige Pad (14) vorzugsweise zwei oder drei Durchgangslöcher umfasst.

8. Einrichtung (10) nach einem der vorstehenden Ansprüche, wobei jeder leitfähige Draht (16, 28, 30; A-M) sich von dem jeweiligen Pad (14) in einer Ebene erstreckt, die durch die elektronische Vorrichtung (12) und die Vielzahl von leitfähigen Pads (14) definiert ist,
und/oder
wobei jeder leitfähige Draht (16, 28, 30; A-M) einen im Wesentlichen geraden Abschnitt, einen gekrümmten Abschnitt (28) und/oder einen gebogenen Abschnitt (30) umfasst.

9. Einrichtung (10) nach einem der vorstehenden Ansprüche, wobei das Beschichtungsmaterial (20) einen Abschnitt jedes leitfähigen Drahts (16, 28, 30; AM) bedeckt, wodurch der leitfähige Draht (16, 28, 30) mit dem leitfähigen Pad (14) verbunden wird.

10. Einrichtung (10) nach einem der vorstehenden Ansprüche, wobei (i) die leitfähigen Pads (14) auf der elektronischen Vorrichtung (12) integriert sind,
oder
wobei (ii) die leitfähigen Pads in direktem Kontakt mit der Textilschnittstelle stehen und/oder von der elektronischen Vorrichtung beabstandet sind,
vorzugsweise wobei (a) die leitfähigen Pads in direktem Kontakt mit der Textilschnittstelle stehen und von der elektronischen Vorrichtung beabstandet sind.

11. Einrichtung (10) nach Anspruch 10 (ii)(a), wobei das Beschichtungsmaterial (20) ein flexibles Material ist, wobei die Porosität der Textilschnittstelle (18) durch eine Vielzahl von Löchern (40) in im Wesentlichen gleichem Abstand voneinander bereitgestellt wird, wobei die Textilschnittstelle (18) ein Netzmaterial ist, und wobei jeder leitfähige Draht (16, 28, 30; A-M) in die Textilschnittstelle (18) eingenäht ist, wobei der Faden der leitfähige Draht (16, 28, 30; A-M) ist, der die erste und die zweite Oberfläche (36, 38) der Textilschnittstelle (18) durchquert, und wobei die leitfähigen Pads in direktem Kontakt mit der Textilschnittstelle stehen und von der elektronischen Vorrichtung beabstandet sind.

12. Einrichtung (10) nach einem der vorstehenden Ansprüche, wobei die elektronische Vorrichtung (12) eine starre Leiterplatte umfasst.

13. Einrichtung (10) nach einem der vorstehenden Ansprüche, wobei die Einrichtung (10) als tragbare freistehende Einrichtung konfiguriert ist und wobei die elektronische Vorrichtung (12) eine elektrische Energiespeichervorrichtung (34) umfasst, die mit einer Ladeschnittstelle (22) verbunden ist,
wobei die Ladeschnittstelle (22) vorzugsweise als zwei Verbindungsschnittstellen (24, 26) ausgebildet ist, die jeweils über einen in die Textilschnittstelle (18) eingenähten leitfähigen Draht (16, 30) mit der elektronischen Vorrichtung (12) verbunden sind,
wobei jede Verbindungsschnittstelle (24, 26) mehr bevorzugt ein Befestigungsmittel umfasst, noch mehr bevorzugt einen Klettverschluss oder einen Druckknopfverschluss.

14. Einrichtung (10) nach einem der vorstehenden Ansprüche, wobei die elektronische Vorrichtung (12) mindestens ein Durchgangsloch umfasst und wobei sich mindestens ein leitfähiger Draht durch das Durchgangsloch erstreckt und die elektronische Vorrichtung (12) an der Textilschnittstelle (18) befestigt.

15. Einrichtung (10) nach einem der vorstehenden Ansprüche, wobei jeder leitfähige Draht (16, 28, 30; A-M) eine im Wesentlichen homogene Außenfläche umfasst und aus einer Vielzahl von mindestens teilweise materialhaftenden miteinander verbundenen leitfähigen Drähten ausgebildet ist.

16. Einrichtung (10) nach einem der vorstehenden Ansprüche, wobei die elektronische Vorrichtung (12) konfiguriert ist, um die biometrischen Sensorsignale zu empfangen, zu verarbeiten und in mehrparametrige biometrische Daten zu speichern, und einen Sender oder Transceiver zum Übertragen der Daten umfasst,
wobei die elektronische Vorrichtung (12) vorzugsweise konfiguriert ist, um drahtlos mit einer zweiten Vorrichtung gekoppelt werden zu können, noch mehr bevorzugt durch Auslesen optischer Informationen, die an der Vorrichtung (10) angebracht sind, auf der zweiten Vorrichtung.

17. Kleidungsstück, vorzugsweise ein T-Shirt, umfassend einen tragbaren Stoff (42) und eine Einrichtung (10) nach einem der vorstehenden Ansprüche, wobei die Textilschnittstelle (18) der Einrichtung in den tragbaren Stoff (42) eingenäht ist, vorzugsweise in einem Bereich, der den Nähten (44) des Kleidungsstücks entspricht.

18. Verfahren zum Herstellen einer Einrichtung zum Verarbeiten von Sensorsignalen, umfassend die Schritte:
- Bereitstellen einer elektronischen Vorrichtung, die eine starre oder flexible Leiterplatte umfasst und konfiguriert ist, um biometrische Sensorsignale eines Benutzers zu empfangen und zu verarbeiten, sowie eine Vielzahl von elektrisch leitfähigen Pads, die mit der elektronischen Vorrichtung verbunden sind, wobei jedes Pad mit einem jeweiligen elektrisch leitfähigen Draht verbunden ist, der konfiguriert ist, um einen Sensor, der sich mit einem anatomischen Bereich eines Benutzers deckt, zu kontaktieren und ein Sensorsignal an die elektronische Vorrichtung bereitzustellen;
- Bereitstellen einer Textilschnittstelle zur Verwendung in einem tragbaren Stoff mit einer ersten Oberfläche und einer zweiten Oberfläche und Umfassen einer Porosität, die eine Fluidkommunikation zwischen der ersten Oberfläche und der zweiten Oberfläche bereitstellt;
- Anordnen und Befestigen der elektronischen Vorrichtung und der Vielzahl von Pads auf der ersten Oberfläche eines Abschnitts der Textilschnittstelle und der Vielzahl von leitfähigen Drähten auf der ersten Oberfläche der Textilschnittstelle; und
- Bereitstellen eines Beschichtungsmaterials über mindestens die elektronische Vorrichtung und die Vielzahl von Pads, das sich von der ersten Oberfläche durch die Textilschnittstelle zu der zweiten Oberfläche erstreckt, wodurch mindestens die elektronische Vorrichtung, die Vielzahl von Pads und der Abschnitt der Textilschnittstelle fluidisch abgedichtet und eingekapselt werden.

19. Verfahren nach Anspruch 18, wobei die bereitgestellte elektronische Vorrichtung ferner eine Batterie zum Bereitstellen elektrischer Energie für die elektronische Vorrichtung (12) umfasst.

20. Verfahren nach Anspruch 18 oder 19, wobei (i) der Schritt des Bereitstellens eines Beschichtungsmaterials das Spritzgießen mit Beschichtungsmaterial einschließt, wobei das Beschichtungsmaterial vorzugsweise ein Elastomer oder mehr bevorzugt silikon basiert ist,
und/oder
wobei (ii) die Textilschnittstelle ein Netzmaterial oder Tüll ist und wobei der leitfähige Draht durch Nähen unter Verwendung des leitfähigen Drahtes als ein Faden an der Textilschnittstelle befestigt ist.

21. Verfahren nach einem der Ansprüche 18 bis 20, wobei das Verfahren eine Einrichtung nach einem der Ansprüche 1 bis 16 bereitzustellen.

22. Verfahren nach Anspruch 20 (ii) oder 21, wobei jedes leitfähige Pad mindestens zwei Durchgangslöcher umfasst und wobei der Schritt des Befestigens der leitfähigen Pads an der Textilschnittstelle umfasst, dass jedes Kontaktpad in die Textilschnittstelle unter Verwendung des jeweiligen leitfähigen Drahts als ein Faden eingenäht wird und sich der leitfähige Draht durch die Durchgangslöcher erstreckt,
vorzugsweise wobei die leitfähigen Pads in die elektronische Vorrichtung integriert sind oder wobei die leitfähigen Pads in direktem Kontakt mit der Textilschnittstelle stehen und/oder von der elektronischen Vorrichtung beabstandet sind.

23. Verfahren nach einem der Ansprüche 18 bis 22, wobei die elektronische Vorrichtung mindestens ein Durchgangsloch umfasst und wobei der Schritt des Befestigens der elektronischen Vorrichtung an der Textilschnittstelle das Annähen der elektronischen Vorrichtung unter Verwendung des Durchgangslochs und unter Verwendung eines leitfähigen Drahtes als ein Faden umfasst.

24. Verfahren zum Herstellen eines Kleidungsstücks, vorzugsweise eines T-Shirts, umfassend die Schritte des Verfahrens nach einem der vorstehenden Ansprüche 18 bis 23 und Bereitstellen eines tragbaren Stoffes und Einnähen der Textilschnittstelle in den tragbaren Stoff, vorzugsweise in einem Bereich, der den Nähten des Kleidungsstücks entspricht.

## Revendications

1. Appareil (10) permettant de traiter des signaux de capteur, comprenant :
- un dispositif électronique (12) comprenant une carte de circuit imprimé rigide ou souple et configuré pour recevoir et traiter les signaux de capteur biométriques d'un utilisateur,
- une pluralité de tampons électriquement conducteurs (14) connectés au dispositif électronique (12), dans lequel chaque tampon (14) est connecté à un fil électriquement conducteur (16, 28, 30 ; A-M) respectif configuré pour entrer en contact avec un capteur correspondant à une région anatomique d'un utilisateur et pour fournir un signal de capteur au dispositif électronique (12), et
- une interface textile (18) destinée à être utilisée dans un tissu portable (42) ayant une première surface (36) et une seconde surface (38) et comprenant une porosité fournissant une communication fluidique entre la première surface (36) et la seconde surface (38), dans lequel le dispositif électronique (12) et la pluralité de tampons (14) sont agencés sur la première surface (36) d'une partie de l'interface textile (18), et la pluralité de fils conducteurs (16, 28, 30 ; A-M) sont agencés sur la première surface (36) de l'interface textile (18) et dans lequel le dispositif électronique (12), la pluralité de tampons (14) et la pluralité de fils conducteurs (16, 28, 30 ; A-M)) sont fixés à l'interface textile (18),
dans lequel l'appareil (10) comprend en outre un matériau de revêtement (20) recouvrant au moins le dispositif électronique (12), la pluralité de tampons (14) et la partie de l'interface textile (18) et s'étendant de la première surface (36) à travers l'interface textile (18) jusqu'à la seconde surface (38), scellant et encapsulant de ce fait de manière fluidique au moins le dispositif électronique (12), la pluralité de tampons (14) et la partie de l'interface textile (18).

2. Appareil (10) selon la revendication 1, dans lequel le dispositif électronique (12) comprend en outre une batterie pour fournir de l'énergie électrique au dispositif électronique (12).

3. Appareil (10) selon la revendication 1 ou 2, dans lequel le matériau de revêtement (20) est un matériau souple, comprenant de préférence du silicone,
et/ou
dans lequel le matériau de revêtement (20) est moulé sur au moins le dispositif électronique (12) et la pluralité de tampons (14).

4. Appareil (10) selon l'une quelconque des revendications précédentes, dans lequel la porosité de l'interface textile (18) est fournie par une pluralité de trous (40) à distance sensiblement égale les uns des autres,
dans lequel l'interface textile (18) est de préférence un matériau en maille, plus préférablement du tulle.

5. Appareil (10) selon l'une quelconque des revendications précédentes, dans lequel chaque fil conducteur (16, 28, 30 ; A-M) est cousu dans l'interface textile (18), dans lequel le filetage est le fil conducteur (16, 28, 30 ; A-M) traversant la première et la seconde surface (36, 38) de l'interface textile (18).

6. Appareil (10) selon la revendication 1, dans lequel le matériau de revêtement (20) est un matériau souple et dans lequel la porosité de l'interface textile (18) est fournie par une pluralité de trous (40) à distance essentiellement égale les uns des autres, ladite interface textile (18) étant un matériau en maille, et dans lequel chaque fil conducteur (16, 28, 30 ; A-M) est cousu dans l'interface textile (18), dans lequel le filetage est le fil conducteur (16, 28, 30 ; A-M) traversant la première et la seconde surface (36, 38) de l'interface textile (18),
de préférence dans lequel le matériau de revêtement comprend du silicone ou est à base de silicone et/ou dans lequel l'interface textile est en tulle.

7. Appareil (10) selon l'une quelconque des revendications 5 ou 6, dans lequel chacun des tampons conducteurs (14) est fixé à l'interface textile (18) par un fil respectif de la pluralité de fils conducteurs (16, 28, 30 ; A-M),
de préférence, dans lequel chaque tampon conducteur (14) comprend au moins un trou traversant et est cousu dans l'interface textile (18) par le fil conducteur respectif (16, 28, 30 ; A-M) s'étendant à travers l'au moins un trou traversant,
dans lequel chaque tampon conducteur (14) comprend plus préférablement deux ou trois trous traversants.

8. Appareil (10) selon l'une quelconque des revendications précédentes, dans lequel chaque fil conducteur (16, 28, 30 ; A-M) s'étend à partir du tampon respectif (14) dans un plan défini parle dispositif électronique (12) et la pluralité de tampons conducteurs (14),
et/ou
dans lequel chaque fil conducteur (16, 28, 30 ; A-M) comprend une partie essentiellement droite, une partie incurvée (28) et/ou une partie courbée (30).

9. Appareil (10) selon l'une quelconque des revendications précédentes, dans lequel le matériau de revêtement (20) recouvre une partie de chaque fil conducteur (16, 28, 30 ; A-M) connectant le fil conducteur (16, 28, 30) au tampon conducteur (14).

10. Appareil (10) selon l'une quelconque des revendications précédentes, dans lequel (i) les tampons conducteurs (14) sont intégrés au dispositif électronique (12),
ou
dans lequel (ii) les tampons conducteurs sont en contact direct avec l'interface textile et/ou sont espacés du dispositif électronique,
de préférence dans lequel (a) les tampons conducteurs sont en contact direct avec l'interface textile et sont espacés du dispositif électronique.

11. Appareil (10) selon la revendication 10 (ii)(a), dans lequel le matériau de revêtement (20) est un matériau souple, dans lequel la porosité de l'interface textile (18) est fournie par une pluralité de trous (40) à distance essentiellement égale les uns des autres, ladite interface textile (18) étant un matériau en maille, et dans lequel chaque fil conducteur (16, 28, 30 ; A-M) est cousu dans l'interface textile (18), dans lequel le filetage est le fil conducteur (16, 28, 30 ; A-M) traversant la première et la seconde surface (36, 38) de l'interface textile (18) et dans lequel les tampons conducteurs sont en contact direct avec l'interface textile et sont espacés du dispositif électronique.

12. Appareil (10) selon l'une quelconque des revendications précédentes, dans lequel le dispositif électronique (12) comprend une carte de circuit imprimé rigide.

13. Appareil (10) selon l'une quelconque des revendications précédentes, dans lequel l'appareil (10) est configuré comme un appareil portable autonome et dans lequel le dispositif électronique (12) comprend un dispositif de stockage d'énergie électrique (34) connecté à une interface de charge (22),
dans lequel l'interface de charge (22) est de préférence formée de deux interfaces de connexion (24, 26), chacune étant connectée au dispositif électronique (12) par le biais d'un fil conducteur (16, 30) cousu dans l'interface textile (18),
dans lequel chaque interface de connexion (24, 26) comprend plus préférablement un moyen de fixation, encore plus préférablement une fixation à boucles et crochets ou un bouton-pression.

14. Appareil (10) selon l'une quelconque des revendications précédentes, dans lequel le dispositif électronique (12) comprend au moins un trou traversant et dans lequel au moins un fil conducteur s'étend à travers ledit trou traversant et fixe le dispositif électronique (12) à l'interface textile (18).

15. Appareil (10) selon l'une quelconque des revendications précédentes, dans lequel chaque fil conducteur (16, 28, 30 ; A-M) comprend une surface extérieure essentiellement homogène et est formé d'une pluralité de fils conducteurs qui sont au moins en partie reliés par une liaison matérielle.

16. Appareil (10) selon l'une quelconque des revendications précédentes, dans lequel le dispositif électronique (12) est configuré pour recevoir, traiter et stocker les signaux de capteur biométrique dans des données biométriques multiparamétriques et comprend un émetteur ou un émetteur-récepteur pour transmettre lesdites données,
dans lequel le dispositif électronique (12) est de préférence configuré pour être apparié sans fil à un second dispositif, plus préférablement en lisant les informations optiques fixées à l'appareil (10) sur ledit second dispositif.

17. Article vestimentaire, de préférence un T-shirt, comprenant un tissu portable (42) et un appareil (10) selon l'une quelconque des revendications précédentes, dans lequel l'interface textile (18) de l'appareil est cousue dans le tissu portable (42), de préférence au niveau d'une zone correspondant aux coutures (44) de l'article vestimentaire.

18. Procédé de fabrication d'un appareil permettant de traiter des signaux de capteur, comprenant les étapes consistant à :
- la fourniture d'un dispositif électronique comprenant une carte de circuit imprimé rigide ou souple et configuré pour recevoir et traiter les signaux de capteur biométriques d'un utilisateur, et une pluralité de tampons électriquement conducteurs connectés au dispositif électronique, dans lequel chaque tampon est connecté à un fil électriquement conducteur respectif configuré pour entrer en contact avec un capteur correspondant à une région anatomique d'un utilisateur et pour fournir un signal de capteur au dispositif électronique ;
- la fourniture d'une interface textile pour une utilisation dans un tissu portable ayant une première surface et une seconde surface et comprenant une porosité fournissant une communication fluidique entre la première surface et la seconde surface ;
- l'agencement et la fixation du dispositif électronique et de la pluralité de tampons sur la première surface d'une partie de l'interface textile, et la pluralité de fils conducteurs sur la première surface de l'interface textile ; et
- la fourniture d'un matériau de revêtement sur au moins le dispositif électronique et la pluralité de tampons et s'étendant de la première surface à la seconde surface en passant par l'interface textile, scellant et encapsulant de ce fait de manière fluidique au moins le dispositif électronique, la pluralité de tampons et la partie de l'interface textile.

19. Procédé selon la revendication 18, dans lequel le dispositif électronique fourni comprend en outre une batterie pour fournir de l'énergie électrique au dispositif électronique (12).

20. Procédé selon la revendication 18 ou 19, dans lequel (i) l'étape consistant à fournir un matériau de revêtement comporte le moulage par injection avec un matériau de revêtement, dans lequel le matériau de revêtement est de préférence un élastomère ou, plus préférablement à base de silicone,
et/ou
dans lequel (ii) l'interface textile est un matériau en maille ou un tulle et dans lequel le fil conducteur est fixé à l'interface textile par couture à l'aide du fil conducteur comme un filetage.

21. Procédé selon l'une quelconque des revendications 18 à 20, dans lequel le procédé fournit un appareil selon l'une quelconque des revendications 1 à 16.

22. Procédé selon la revendication 20 (ii) ou 21, dans lequel chaque tampon conducteur comprend au moins deux trous traversants et dans lequel l'étape consistant à fixer les tampons conducteurs à l'interface textile comprend la couture de chaque tampon de contact dans l'interface textile à l'aide du fil conducteur respectif comme un filetage et en prolongeant le fil conducteur à travers les trous traversants,
de préférence, dans lequel les tampons conducteurs sont intégrés au dispositif électronique ou dans lequel les tampons conducteurs sont en contact direct avec l'interface textile et/ou sont espacés du dispositif électronique.

23. Procédé selon l'une quelconque des revendications 18 à 22, dans lequel le dispositif électronique comprend au moins un trou traversant et dans lequel l'étape consistant à fixer le dispositif électronique à l'interface textile comprend la couture du dispositif électronique à l'aide dudit trou traversant et à l'aide d'un fil conducteur comme un filetage.

24. Procédé de fabrication d'un vêtement, de préférence un T-shirt, comprenant les étapes du procédé selon l'une quelconque des revendications précédentes 18 à 23 et la fourniture d'un tissu portable et la couture de l'interface textile dans le tissu portable, de préférence au niveau d'une région correspondant aux coutures du vêtement.
